# EUROPEAN PATENT SPECIFICATION

(11) **EP 0 498 476 B1**
(45) Date of publication and mention of the grant of the patent: **27.03.1996**
(21) Application number: 92200035.1
(22) Date of filing: 07.01.1992
(51) Int. Cl.: A61M 25/00, A61B 17/22

(54) **Angiographic/dilatation catheter**
Angiographie-/Dilatationskatheter
Cathéter d'angiographie et/ou dilatation

(30) Priority: 05.02.1991 NL 9100200
(43) Date of publication of application: 12.08.1992
(73) Proprietor: CORDIS EUROPA N.V., NL-9301 LJ Roden (NL)
(72) Inventor: Qureshi, Shakeel Ahmed, London SE1 9RT (GB); Rosenthal, Eric, London SE1 9RT (GB); Persaud, Deodat, London SE24 0NP (GB); Griep, Wilhelmus Antonius Maria, NL-9301 PK Roden (NL)
(74) Representative: 't Jong, Bastiaan Jacobus

(56) References cited:
- EP-A- 0 265 866
- EP-A- 0 335 022
- EP-A- 0 347 098
- EP-A- 0 360 582
- EP-A- 0 369 383
- EP-A- 0 437 248

## Description

The invention relates to an angiographic catheter comprising a flexible tube-like basic body with a central channel and a sheath having an embedded reinforcing layer woven from stainless steel wire. Arranged at a rear end are connecting means for connecting liquid supply means to the central channel.

Such a catheter is generally known see for example EP-A-0 369 383, and is considered to represent the closest prior art with respect to the present invention. Due to the construction with a woven reinforcing layer such a catheter can have a small diameter and still be easily maneuverable. To be able to maneuver a catheter well it has to be torsionally rigid and not have a rapid tendency to form sharp edges, while nevertheless being sufficiently pliant. Known angiographic catheters have at their leading end a determined curvature in order to be able to introduce this leading end and particularly the outlet of the central channel into the body of a patient in a desired position.

The invention provides an angiographic catheter which can moreover be used as dilatation catheter. The angiographic catheter according to the invention comprises a tube-like end part connected to the basic body at the opposite leading end with a continuous and smooth transition, wherein the leading end of the catheter has a curvature and the end part has on the inside a channel connecting to the central channel and having a diameter decreasing regularly from the diameter of the central channel to a smaller diameter of a guide channel close to the extremity and the end part is tapered conically on the outside towards the extremity to substantially the diameter of the guide channel and is given a smooth finish. This makes it possible to use the angiographic catheter to remedy total and sub-total vascular and valvular obstructions. Due to the good maneuverability of the catheter the extremity thereof can be moved accurately to a position close to the obstruction. With a re-canalization wire which can be guided precisely in the guide channel on the leading end a small opening can then be made in the obstruction. After the end of the recanalization wire has passed through the obstruction, this is further widened by pushing the catheter therethrough, wherein the re-canalization wire serves as guide for the catheter. As a result of the lengthwise stiffness of the angiographic catheter sufficient pressure force can be exerted for widening of the obstruction.

The catheter is then left in position, the recanalization wire is exchanged for a guide wire and the catheter can be withdrawn. A per se known balloon catheter is then inserted over the guide wire. The obstruction is widened by the angiographic catheter so far that such a known balloon catheter can move through the obstruction. This obstruction is subsequently further widened in a second step through the known action of the balloon catheter.

It has been found that the operation described can be realized with a braided angiographic catheter of very small diameter, such as a 1.33 mm (French 4) diameter. At such a small diameter the catheter according to the invention is suitable for a pulmonary valve dilatation treatment in the case of congenital pulmonic valve atresia of new-born babies. Alternative applications are the treatment of vascular obstructions like aortic coarctations and high grade stenoses in the coronary arteries.

The invention also relates to and provides an assembly of a catheter as specified above and a re-canalization wire, wherein the diameter of the guide channel is substantially equal to the outer diameter of the re-canalization wire.

The invention will be further elucidated in the following description of an embodiment and a possible application of the catheter according to the invention.

Fig. 1 shows a general view of a catheter according to the invention.

Fig. 2 shows a partly broken away view on a larger scale of the catheter of fig. 1.

Fig. 3-11 show an application of the catheter according to the invention.

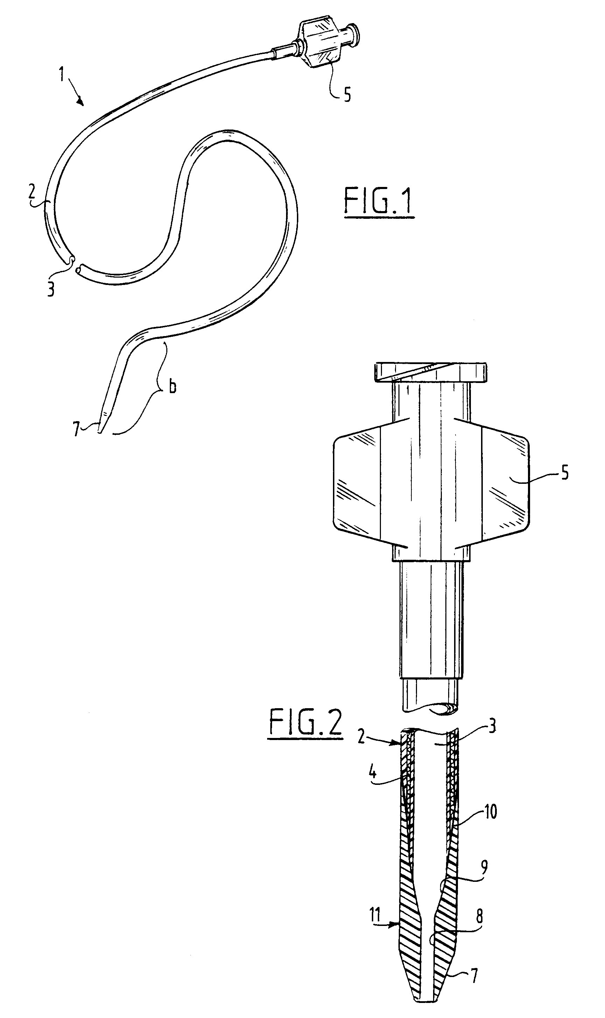

The angiographic catheter 1 according to the invention shown in fig. 1 comprises a flexible tube-like basic body 2 with a central channel 3 and a sheath having an embedded reinforcing layer 4 woven from stainless steel wire, as shown in fig. 2.

The basic body 2 is provided on the one end with connecting means 5 for connecting liquid supply means in the form of a per se known Luer fitting to the central channel.

The opposite leading end 6 of catheter 1 has a specific curvature which is adjusted in known manner to the place in the body of the patient where this leading end has to be positioned. The shape shown in fig. 1 is a so-called multi-purpose shape.

The catheter 1 comprises at the leading end an end part 11 into which the basic body 2 transposes smoothly. The end part 11 which is formed for instance in a mould is joined to the basic body 2 along a tapered portion thereof using per se known techniques such as for instance glueing or welding.

On the inside the end part 11 has a channel connecting to the central channel 3 and comprising close to the extremity of the catheter a guide channel 8 with a substantially constant diameter that is smaller than the diameter of central channel 3. The channel of the end part 11 has a curving portion 9 formed such that the channel transposes uniformly from the diameter of the central channel 3 to the smaller diameter of the guide channel 8.

On the outside the end part is provided towards the extremity with a conical tapering 7 converging to substantially the diameter of guide channel 8. The tapering 7, and in particular the transition to the guide channel 8, is given a smooth finish.

In a preferred embodiment of the catheter according to the invention the diameter of the guide channel 8 is virtually equal to 0.45 mm (0.018 inch). This the diameter of typically used re-canalization wires such as hot-tip recanalization laser wires, photo-ablation laser wires or mechanical re-canalization wires and guide wires. In the use of the catheter according to the invention as described hereinafter such a wire can thus slide with precise fitting into the guide channel.

For the above described treatment of new-born babies a catheter according to the invention is preferably used with a 1.33 mm (French 4) diameter. With such a catheter the above mentioned treatment of new-born babies can be performed.

A suitable shape of the conical tapering 7 is achieved with a tapering to a diameter of 0.67-1 mm (2-3 French), whereafter the outermost 0.5 mm is rounded off smoothly to the diameter of the guide channel. This prevents the occurrence of a sharp end edge which could lead to injury, while the intended dilatation treatment can be properly performed with such a shape. In the case of a French 4 catheter the tapering extends over a distance of roughly 5 mm.

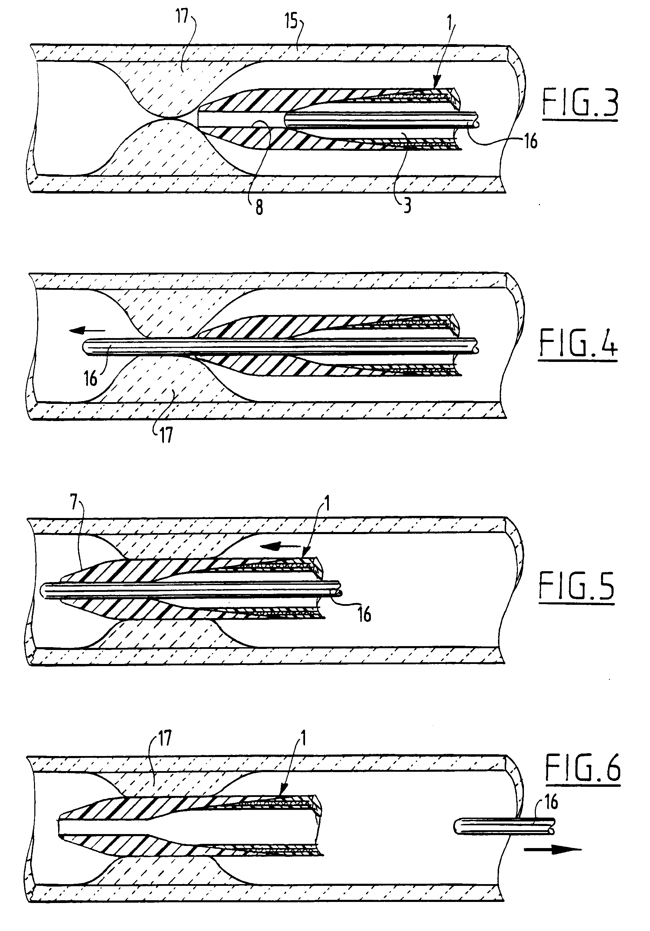

With reference to fig. 3-11 a possible application of the angiographic/dilatation catheter according to the invention will now be described.

Fig. 3 shows schematically an obstruction 17 in a body vessel 15. As previously noted, this may for instance be a pulmonary atresia of the heart of a new-born baby.

In order to relieve the obstruction 17, i.e. to provide a passage, the catheter 1 according to the invention is first maneuvered with its extremity to a position close to the obstruction 17. This takes place in typical manner in a catheter laboratory where, using angiographic techniques, the area where the extremity of the catheter 1 is situated is made visible on the screen of an X-ray apparatus. The specific form of the leading end 6 of the catheter assists in the maneuvering of the catheter to the correct position.

When the catheter has been placed in position in the manner shown in fig. 3, a re-canalization wire 16 is inserted via the central channel 3. This re-canalization wire 16 has an outer diameter practically corresponding to the inner diameter of the guide channel 8 of the catheter. The recanalization wire 16 can for example be a hot-tip laser wire, the leading tip of which can be heated through energy transport internally in the wire 16 using laser light. Due to the guiding of the catheter 1 the re-canalization wire 16 is held in the correct position relative to the obstruction 17 and, when it is activated and moved slowly forward in the direction of the arrow as shown in fig. 4, the end of the wire 16 can pass through the obstruction 17.

After it has been determined that the wire 16 has indeed passed through obstruction 17 the catheter 1 is pressed in the direction of the arrow in fig. 5. As a result of the structure of the catheter with woven reinforcing layer 4, it has sufficient pressure rigidity to enable the tapering 7 to be pressed through the obstruction 17. Herein, the wire 16 functions in turn as guiding for the catheter 1. Using the catheter 1 the obstruction 17 is thus further widened to a suitable diameter for a further treatment with dilatation balloon catheters.

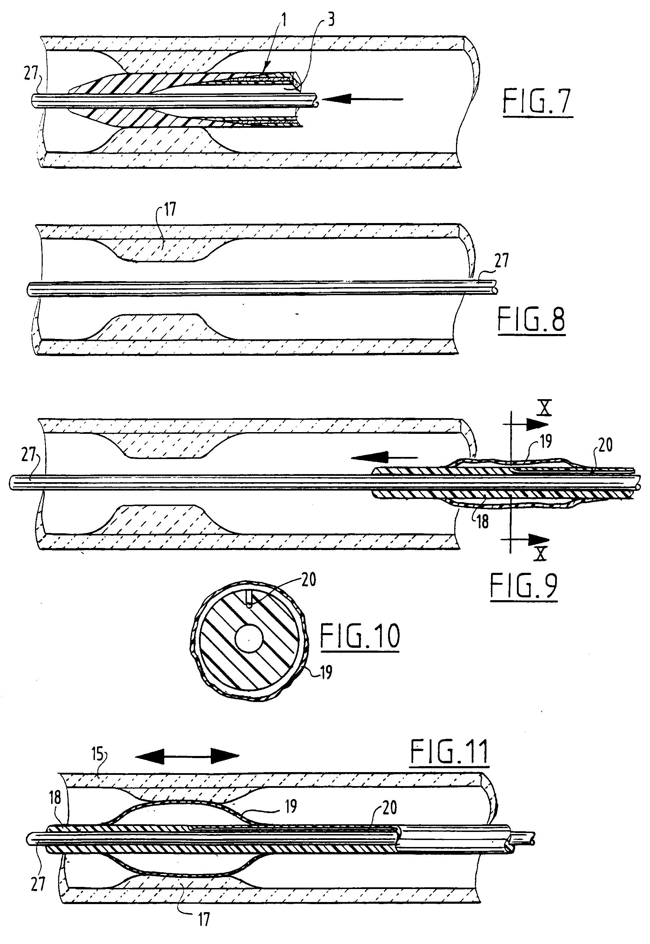

For this further treatment the re-canalization wire 16 is first withdrawn as shown in fig. 6. The catheter 1 remains in place with the end part pushed through the obstruction 17. A normal guide wire 27 is subsequently pushed through the central channel of catheter 1, this wire having a diameter roughly equal to that of the re-canalization wire 16. This step of the treatment is shown in fig. 7. Once the guide wire 27 has been placed in position through the remaining obstruction 17, the catheter 1 can be withdrawn.

With the guide wire 27 as guiding a per se known balloon catheter 18 is then inserted until the balloon 19 is situated at the location of the residual obstruction 17. The balloon catheter 18 is provided in known manner with an extra channel 20 through which a liquid can be pumped into the balloon 19 in order to bring about inflation thereof.

With the balloon 19 at the location of the obstruction 17 liquid is thus supplied via the channel 20, whereby the inflating balloon 19 presses the obstruction 17 further open. This dilatation treatment using the balloon catheter 18 can if necessary be carried out in a number of steps, wherein slightly larger balloon catheters are used each time.

The catheter according to the invention is of course not limited to a catheter with the said specific dimensions. It has been found however that the catheter according to the invention with the particularly small French 4 diameter can function successfully in the specially difficult field of treatment of new-born babies.

## Claims

1. Angiographic catheter (1) comprising a flexible tube-like basic body (2) with a central channel (3) and a sheath having an embedded reinforcing layer (4) woven from stainless steel wire, connecting means (5) at a rear end for connecting liquid supply means to the central channel, characterised by a tube-like end part (11) connected to the basic body at the opposite leading end with a continuous and smooth transition therefrom, wherein the leading end of the catheter has a curvature and the end part has on the inside a channel (9) connecting to the central channel and having a diameter decreasing smoothly from the diameter of the central channel to a smaller diameter of a guide channel (8) close to the extremity, and on the outside (7) the end part is tapered conically towards the extremity to substantially the diameter of the guide channel and is given a smooth finish.

2. Catheter as claimed in claim 1, wherein the diameter of the guide channel is practically 0.45 mm (0.018 inch).

3. Catheter as claimed in claim 2, wherein the end part is conically tapered to a diameter of 0.67-1 mm (2-3 French) and the final 0.5 mm is rounded off smoothly to the diameter of the guide channel.

4. Catheter as claimed in any of the foregoing claims having a 1.33 mm (French 4) diameter.

5. Assembly of a catheter as claimed in any of the foregoing claims and a re-canalisation wire (16), such as a hot-tip laser wire, a photo-ablation laser wire or a mechanical re-canalization wire, wherein the diameter of the guide channel is substantially equal to the outer diameter of the re-canalization wire.

## Patentansprüche

1. Angiographik-Katheter (1), der einen flexiblen röhrchenförmigen Basiskörper (2) mit einem zentralen Kanal (3) und einer Höhle, die eine eingebettete Verstärkungsschicht (4) aufweist, die aus rostfreiem Stahldraht gewebt ist, und Anschlußeinrichtungen (5) am Hinterende aufweist, um eine Flüssigkeitsverteileinrichtung zum zentralen Kanal anzuschließen,
**gekennzeichnet durch,**
einen röhrchenförmigen Endabschnitt (11), der mit dem Basiskörper an dem abgewandten Vorderende mit einem kontinuierlichen und sanften Übergang davon verbunden ist, wobei das Vorderende des Katheters eine Krümmung aufweist und der Endabschnitt an der Innenseite einen Kanal (9) aufweist, der mit dem zentralen Kanal verbunden ist und einen Durchmesser aufweist, der vom Durchmesser des zentralen Kanales zum geringeren Durchmesser eines Führungskanales (8) in der Nähe der äußeren Enden sanft abnimmt, und wobei an der Außenseite (7) der Endabschnitt konisch in Richtung der äußeren Enden auf im wesentlichen den Durchmesser des Führungskanales zugespitzt ist und einen glatten Abschluß bildet.

2. Katheter nach Anspruch 1,
bei dem der Durchmesser des Führungskanales praktischerweise 0,45 mm (0.018 inch) beträgt.

3. Katheter nach Anspruch 2,
bei dem der Endabschnitt konisch auf einen Durchmesser von 0,67 bis 1 mm (2 bis 3 French) zugespitzt ist und bei dem die letzten 0,5 mm sanft bis zum Durchmesser des Führungskanales abgerundet sind.

4. Katheter nach einem der vorhergehenden Ansprüche,
der einen Durchmesser von 1,33 mm (French 4) aufweist.

5. Anordnung aus einem Katheter nach einem der vorhergehenden Ansprüche
und einem Rekanalisationsdraht (16), wie z.B. ein Laserdraht mit heißer Spitze, ein Photo-Ablations-Laserdraht oder ein mechanischer Rekanalisationsdraht, wobei der Durchmesser des Führungskanales im wesentlichen gleich dem Außendurchmesser des Rekanalisationsdrahtes ist.

## Revendications

1. Cathéter angiographique (1) comprenant un corps de base tubulaire flexible (2) présentant un canal central (3) et une gaine ayant une couche de renforcement noyée (4) tissée en fils d'acier inoxydable, des moyens de raccordement (5) situés à une extrémité arrière pour raccorder des moyens d'alimentation en liquide au canal central, caractérisé par une partie terminale tubulaire (11) raccordée au corps de base à l'extrémité de tête opposée, avec une transition continue et lisse à partir de cette tête, dans lequel l'extrémité de tête du cathéter possède une courbure et la partie terminale possède, sur la surface intérieure, un canal (9) qui se raccorde au canal central et présente un diamètre gui décroît uniformément dudit diamètre du canal central à un plus petit diamètre d'un canal de guidage (8) proche de l'extrémité et, sur la surface extérieure (7), la partie terminale se rétrécit en cône vers l'extrémité à peu près jusqu'au diamètre du canal de guidage et a reçu une finition lisse.

2. Cathéter selon la revendication 1, dans lequel le diamètre du canal de guidage est pratiquement de 0,45 mm (0,018 pouce).

3. Cathéter selon la revendication 2, dans lequel la partie terminale se rétrécit en cône jusqu'à un diamètre de 0,67-1 mm (2-3 French) et la partie finale, de 0,5 mm, est arrondie progressivement pour atteindre le diamètre du canal de guidage.

4. Cathéter selon une quelconque des revendications précédentes, ayant un diamètre de 1,3 mm (4 French).

5. Ensemble d'un cathéter selon une quelconque des revendications précédentes et d'un fil de recanalisation (16) tel qu'un fil laser à pointe chaude, un fil laser de photoablation ou un fil de recanalisation mécanique, dans lequel le diamètre du canal de guidage est sensiblement égal au diamètre extérieur du fil de recanalisation.
